# EUROPÄISCHE PATENTSCHRIFT

(11) **EP 0 667 336 B1**
(45) Veröffentlichungstag und Bekanntmachung des Hinweises auf die Patenterteilung: **20.05.1998**
(21) Anmeldenummer: 95100787.1
(22) Anmeldetag: 20.01.1995
(51) Int. Cl.: C07C 68/00, C07C 69/96

(54) **Verfahren zur kontinuierlichen Herstellung von Diarylcarbonaten**
Process for the continuous preparation of diaryl carbonates
Procédé de préparation contenue de carbonates de diaryle

(30) Priorität: 02.02.1994 DE 4403075
(43) Veröffentlichungstag der Anmeldung: 16.08.1995
(73) Patentinhaber: BAYER AG, 51368 Leverkusen (DE)
(72) Erfinder: Buysch, Hans-Josef, Dr., D-47809 Krefeld (DE); Hesse, Carsten, Dr., D-47800 Krefeld (DE); Rechner, Johann, Dr., D-47800 Krefeld (DE); Schomäcker, Reinhard, Dr., D-51381 Leverkusen (DE); Wagner, Paul, Dr., D-40597 Düsseldorf (DE); Kaufmann, Dieter, Prof. Dr., D-38640 Goslar (DE)

(56) Entgegenhaltungen:
- EP-A- 0 413 215
- EP-A- 0 460 732
- DE-A- 2 815 512

## Beschreibung

Die vorliegende Erfindung betrifft ein Verfahren zur Herstellung von Diarylcarbonaten durch Umsetzung einer aromatischen Hydroxyverbindung (z.B. Phenol) mit Kohlenmonoxid und Sauerstoff in Gegenwart eines Katalysators, eines Cokatalysators, eines quartären Salzes und einer Base, das dadurch gekennzeichnet ist, daß man Wasser kontinuierlich mit dem Reaktionsgas durch Strippen entfernt.

Es ist bekannt, organische Carbonate durch oxidative Umsetzung einer aromatischen Hydroxyverbindung mit Kohlenmonoxid in Gegenwart eines Edelmetall-Katalysators und eines Cokatalysators herzustellen (DE-OS 2 738 437). Als Edelmetalle werden die Elemente der Gruppe VIIIb vorgeschlagen, wobei bevorzugt Palladium eingesetzt wird. Während der Reaktion wird diese Palladium(II)-Spezies zu Palladium(0) reduziert und durch Sauerstoff mit Hilfe eines Cokatalysators wieder zu Palladium(II) oxidiert. Als Cokatalysatoren können verschiedene Mangan-oder Kobaltsalze in unterschiedlichen Oxidationsstufen eingesetzt werden. Neben diesen Cokatalysatoren werden eine Base, ein quartäres Ammonium-Salz und ein Trockenmittel eingesetzt. Als Lösungsmittel wird bevorzugt Methylenchlorid verwendet.

Nachteile dieses Verfahrens sind neben dem Einsatz des leichtflüchtigen und bei Brand in Phosgen umwandelbaren Methylenchlorids als Lösungsmittel, das einen hohen sicherheitstechnischen Aufwand erfordert und kostenintensiv zurückgewonnen werden muß, lange Reaktionszeiten und die damit verbundenen schlechten Raum-Zeit-Ausbeuten. Für eine technische Umsetzung erweist sich jedoch die ungenügende Reproduzierbarkeit als entscheidender Nachteil, da man von Ansatz zu Ansatz bei gleicher Verfahrensweise ganz unterschiedliche Resultate, ja sogar völliges Versagen der Katalyse erhalten kann.

In EP 350 700 wird die Verwendung von verschiedenen Kobaltsalzen, bevorzugt Kobaltacetat als Cokatalysator vorgeschlagen. Neben diesem Cokatalysator wird auch die Verwendung erheblicher Mengen verschiedener Chinone oder Hydrochinone als Elektronen-Transfer-Katalysator vorgeschlagen. Die Abtrennung des Elektronen-Transfer-Katalysators aus dem Reaktionsgemisch erfordert in diesem Verfahren einen erheblichen Aufwand. Zudem stellen Hydrochinone eine zusätzliche aromatische bifunktionelle Hydroxyverbindung dar, die in gleicher Weise zu Carbonaten umgesetzt werden kann. Die Abtrennung der auf diese Weise gebildeten Nebenprodukte kann nur mit großem Aufwand erreicht werden. Eine Wiedergewinnung des eingesetzten Elektronen-Transfer-Katalysators ist nicht möglich. Die Bildung dieser Nebenprodukte senkt die Selektivität und damit die Wirtschaftlichkeit dieses Prozesses erheblich. Auch in dieser Anmeldung wird das Problem der ungenügenden Reproduzierbarkeit nicht gelöst.

In den genannten Verfahrensvorschlägen ist der Zusatz von Molsieb zur Wasserbindung erforderlich. In Abwesenheit von Molsieb fällt der erzielbare Umsatz deutlich geringer aus, da gebildetes Carbonat durch gleichzeitig gebildetes Reaktionswasser hydrolysiert wird. Allerdings macht die Verwendung von Molsieben eine technische Nutzung des Verfahrens unattraktiv, da für eine effektive Abtrennung des Wassers aus der Flüssigphase große Mengen Molsieb (100 bis 500 % Überschuß) benötigt werden, die unter hohem Aufwand regeneriert werden müssen.

Der Einsatz von Kohlendioxid als Trockenmittel wird in EP 450 442 vorgeschlagen. Dazu werden dem Reaktionsgas, das aus Sauerstoff und Kohlenmonoxid besteht, ca. 30 bis 35 % Kohlendioxid zugesetzt. Auch hier werden erhebliche Mengen Chinone/Hydrochinone als Elektronen-Transfer-Katalysator zugesetzt. Die durch die Zugabe der Elektronen-Transfer-Katalysatoren auftretenden Probleme bezüglich der Nebenproduktbildung, der schlechten Selektivität und des Verlustes an Katalysator sind, wie oben bereits beschrieben, auch hier gravierende Nachteile. Darüberhinaus stellt die Verwendung von Kohlendioxid als Trockenmittel keinen Vorteil dar. Dies wird an zwei wesentlichen Punkten deutlich:

Erstens ist Kohlendioxid zur Trocknung des Reaktionsgemisches nur schlecht geeignet. Dies geht deutlich aus dem Vergleich der Beispiele in EP 450 442 (Trockenmittel Kohlendioxid) und EP 350 700 (Trockenmittel Molsieb) hervor. So wird in Gegenwart des Trockenmittels Kohlendioxid nach 5 Stunden Reaktionszeit nur etwa ein Drittel der Ausbeute erzielt, die in der gleichen Zeit mit Molsieb als Trockenmittel erhalten wird. Wegen seiner geringen Effektivität muß Kohlendioxid in großen Mengen (ca. 35 % des Reaktionsgases) zugesetzt werden. Dies führt zu einer starken Verdünnung des Reaktionsgases, wodurch die erzielbaren Raum-Zeit-Ausbeuten dramatisch gesenkt werden. Schließlich muß bei einer Kreisführung des Reaktionsgases, wie es die technische Umsetzung dieses Verfahrens erfordert, eine Anreicherung des Kohlendioxids im Gasstrom durch aufwendige Verfahren verhindert werden. Dies erfordert zusätzlich einen hohen apparativen Aufwand und damit verbunden hohe Kosten, die eine wirtschaftliche Nutzung dieses Verfahrens schon aus diesem Grund unmöglich machen.

Zweitens verhält sich Kohlendioxid nicht inert gegenüber den Reaktionskomponenten. Basen wie beispielsweise Natriumhydroxid, können mit Kohlendioxid zu unlöslichen Produkten reagieren. Dadurch kann das Katalysatorsystem soweit desaktiviert werden, daß keine Carbonatbildung mehr stattfindet. Eine gleichzeitige Verwendung von Kohlendioxid als Trockenmittel und Base im Katalysatorsystem ist also unmöglich.

In JP 04/257 546 wird ein Verfahren beschrieben, bei dem die Reaktion mit Kohlenmonoxid in Gegenwart eines Edelmetall-Katalysators und eines quartären Salzes durch kontinuierliches Einspeisen in eine Destillationskolonne durchgeführt wird. Das Reaktionswasser wird kontinuierlich abdestilliert.

Nachteilig bei diesem Verfahren ist, daß man zur Entfernung des Reaktionswassers in einer Destillationskolonne arbeiten muß, die aufgrund ihrer Konstruktion nur kurze Verweilzeiten ermöglicht. Die mit diesem Verfahren realisierbaren Raum-Zeit-Ausbeuten sind dem zufolge mit nur 17,8 g/l·h sehr niedrig.

Dieser Verfahrensnachteil wiegt besonders schwer vor dem Hintergrund des extrem hohen Katalysatoraufwandes, der für diese Raum-Zeit-Ausbeuten notwendig ist. So werden im Beispiel 1 der Anmeldeschrift bei einer Belastung von 182 g Phenol pro Stunde insgesamt 3 g/h Palladiumverbindung und 14,4 g/h quartäres Ammoniumsalz eingesetzt. Bei einer angegebenen Ausbeute von 35 g Diphenylcarbonat pro Stunde werden also in einer Stunde nur 16,3 Katalysatorzyklen erzielt. Dies bedeutet, daß zur Herstellung eines Kilogramms Diphenylcarbonat nach diesem Verfahren mindestens 30 g reines Palladium (entsprechen 90 g Palladiumverbindung) benötigt werden. Dies bedingt sehr hohe Investitionskosten für den Katalysator und zusätzlich einen hohen Aufwand für die Wiedergewinnung des Edelmetalls. Eine wirtschaftliche Nutzung des Verfahrens ist somit unmöglich. Die Verwendung großer Mengen Halogenide bei hohen Temperaturen von 150 bis 180°C, wie sie in diesem Verfahren erforderlich sind, führt zu großen Korrosionsproblemen, die einen hohen apparativen Aufwand bedingen. Dem Fachmann ist außerdem bekannt, daß unter den angegebenen Reaktionsbedingungen das bevorzugt eingesetzte Iodid des quartären Salzes nicht stabil ist und in erheblichem Maße zu Iod oxidiert wird. Dies führt zu großen Verlusten des quartären Salzes und zur Bildung von Nebenprodukten, was die Selektivität und damit die Wirtschaftlichkeit dieses Verfahrens stark beeinträchtigt.

In JP/04 261 142 wird ein Verfahren beschrieben, bei dem wie in JP 04/257 546 gearbeitet wird, mit dem Unterschied, daß an die Destillationskolonne zur Verweilzeiterhöhung zusätzliche Reaktoren angebracht wurden. Die oben angeführten Probleme mit Korrosion, Katalysatoraufwand und Verlust an quartärem Salz sowie den damit verbundenen Nebenreaktionen sind auch in dieser Anmeldung nicht gelöst. Die vorgeschlagene Apparatur bringt ebenfalls keine Vorteile. Die Verweilzeit wird durch die zusätzlichen Reaktoren vergrößert. Die vorgeschlagene Konstruktion führt aber zu einer erheblichen Rückvermischung innerhalb der Apparatur, so daß Nebenreaktionen in verstärktem Maße ablaufen können, wodurch die Selektivität zurückgeht. So wird im Ausführungsbeispiel 1 von JP 04/261 142 eine Selektivität von nur 97 % gegenüber 99 % im vergleichbaren Beispiel 1 von JP 04/257 546 erzielt. Die mit diesem Verfahren erzielbaren Raum-Zeit-Ausbeuten sind mit ca. 9 g/l·h noch geringer als bei JP 04/257 546. Durch die zusätzlich angebrachten Reaktoren wird eine effektive Entfernung des Reaktionswassers unmöglich. Bei der vorgeschlagenen Verfahrensweise wird das während der Reaktion in den Reaktoren gebildete Reaktionswasser nämlich erst nachträglich in der Destillationskolonne entfernt. Unter den Reaktionsbedingungen wird in den Reaktoren gebildetes Carbonat hydrolytisch wieder gespalten, wodurch nur sehr niedrige Umsätze erzielbar sind.

In keiner der genannten Anmeldung wird das Problem der ungenügenden Reproduzierbarkeit gelöst, so daß insgesamt ein technisch umsetzbares Verfahren bisher nicht zur Verfügung steht.

Es bestand daher die Aufgabe, ein Verfahren zu finden, das es erlaubt, die Synthese aromatischer Carbonate unter kontinuierlicher Entfernung des entstehenden Reaktionswassers mit hoher Raum-Zeit-Ausbeute unter wirtschaftlichen, technisch realisierbaren und reproduzierbaren Bedingungen durchzuführen.

Überraschend wurde nun gefunden, daß man die kontinuierliche Herstellung von Diarylcarbonaten durch oxidative Umsetzung einer aromatischen Hydroxyverbindung in Gegenwart eines Edelmetall-Katalysators, eines Cokatalysators, eines quartären Salzes und einer Base mit hoher Raum-Zeit-Ausbeute und sehr niedrigem Katalysatoraufwand durchführen kann, wenn man das entstehende Reaktionswasser kontinuierlich mit überschüssigen Reaktionsgas aus der Reaktionslösung abstrippt.

Die Erfindung betrifft demnach ein Verfahren zur Herstellung eines organischen Carbonats der Formel (I)

R-O-CO-O-R (I)

in der
- R: ein gegebenenfalls substituiertes C₆-C₁₂-Aryl, bevorzugt ein gegebenenfalls substituiertes C₆-Aryl, besonders bevorzugt Phenyl bedeutet,
durch Umsetzung einer aromatischen Hydroxyverbindung der Formel (II),

R-O-H (II),

worin
- R: die oben angegebene Bedeutung hat,
mit Kohlenmonoxid und Sauerstoff in Gegenwart eines Edelmetall-Katalysators, der in Gegenwart eines quartären Salzes durch Behandlung mit Kohlenmonoxid in flüssiger Phase bei erhöhter Temperatur aus einer Verbindung eines Edelmetalls der Gruppe VIII b des Periodensystems der Elemente (Mendelejew) hergestellt wurde, eines Cokatalysators, eines quartären Salzes und einer Base, das dadurch gekennzeichnet ist, daß man das Reaktionswasser durch überschüssiges Reaktionsgas aus dem Reaktionsgemisch entfernt.

Das Reaktionsgas, bestehend aus Kohlenmonoxid, Sauerstoff und einem Inertgas, wird hierzu in einer Menge von 1 bis 100.000 Nl pro Liter Reaktionslösung, bevorzugt 5 bis 50.000 Nl pro Liter Reaktionslösung und besonders bevorzugt 10 bis 10.000 Nl pro Liter Reaktionslösung eingeleitet.

Die Zusammensetzung der Reaktionsgase Kohlenmonoxid und Sauerstoff kann in weiten Konzentrationsgrenzen variiert werden. Es wird jedoch zweckmäßigerweise ein CO:O₂-Molverhältnis (normiert auf CO) von 1:(0,001-1,0) bevorzugt 1:(0,01-0,5) und besonders bevorzugt von 1:(0,02-0,3) eingestellt. Der Sauerstoffpartialdruck ist bei diesen Molverhältnissen groß genug, um hohe Raum-Zeit-Ausbeuten erreichen zu können und gleichzeitig keine explosionsfähigen Kohlenmonoxid/Sauerstoff-Gasgemische bilden zu können. Die Reaktionsgase unterliegen keinen besonderen Reinheitsanforderungen, so kann Synthesegas als CO-Quelle und Luft als O₂-Träger dienen, es ist jedoch darauf zu achten, daß keine Katalysatorgifte wie z.B. Schwefel oder dessen Verbindungen eingetragen werden. In der bevorzugten Ausführungsform des erfindungsgemäßen Verfahrens werden reines CO und reiner Sauerstoff verwendet. In einer weiteren bevorzugten Ausführungsform des erfindungsgemäßen Verfahrens werden CO und Sauerstoff unabhängig voneinander dosiert. Die Dosierung des Sauerstoffs kann hierbei gegebenenfalls zusammen mit Inertgas erfolgen. Im Falle der Verwendung einer Reaktorkaskade anstelle eines Einzelreaktors erfolgt die separate Sauerstoffdosierung bevorzugt so, daß in jedem der Reaktoren die optimale Sauerstoffkonzentration gewährleistet ist.

Inerte Bestandteile der Reaktionsgase im erfindungsgemäßen Verfahren können Stickstoff, Wasserstoff, Edelgase sowie unter Reaktionsbedingungen beständige organische Verbindungen, die mit Wasser ein Azeotrop bilden, sein. Die Konzentration an Inertgas im Reaktionsgas kann 0-30 Vol-%, bevorzugt 0-15 Vol-%, besonders bevorzugt 0-5 Vol-% betragen. Die Konzentration 0 Volumen-% stellt den Spezialfall des bevorzugten inertgas-freien Zustands dar.

Durch ein im Abgasstrom befindliches Trennorgan, wie z.B. Dephlegmator, Destillationskolonnen mit Böden oder Packung und weitere dem Fachmann bekannte Apparate, kann der größte Teil des mitgerissenen Phenols bzw. Lösungsmittels wieder in den Reaktor zurückgeführt werden. Das mit Wasser angereicherte überschüssige Reaktionsgas wird in der bevorzugten Ausführungsform nach der Wasserabtrennung wieder in den Reaktor zurückgeführt. Die Wasserabtrennung aus dem Reaktionsgas erfolgt nach dem Stand der Technik, z.B. durch Adsorption, Absorption oder bevorzugt durch Abkühlung des unter Druck stehenden Gases und Kondensation des Wassers (Ullmann's Encyclopedia of Industrial Chemistry, 5. Auflage, Volume A5, Seiten 203ff, Weinheim 1986; Ullmann's Encyclopedia of Industrial Chemistry, 5. Auflage, Volume A12 (1989), Seiten 169ff).

Bei den in das erfindungsgemäße Verfahren einzusetzenden aromatischen Hydroxyverbindungen handelt es sich beispielsweise um Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol und 2-Naphthol, bevorzugt um Phenol. Allgemein handelt es sich im Falle einer Substitution der aromatischen Hydroxyverbindung um 1 oder 2 Substituenten in der Bedeutung von C₁-C₄-Alkyl, C₁-C₄-Alkoxy, Fluor, Chlor oder Brom.

Für das erfindungsgemäße Verfahren können beliebige sowohl organische als auch anorganische Basen oder Mischungen derselben verwendet werden. Als Beispiele für anorganische Basen seien, ohne das erfindungsgemäße Verfahren einzuschränken, Alkalimetallhydroxide und -carbonate, -carboxylate oder andere Salze schwacher Säuren sowie Alkalisalze von aromatischen Hydroxyverbindungen der Formel (II), z.B. Alikalimetallphenolate, genannt. Selbstverständlich können in das erfindungsgemäße Verfahren auch die Hydrate von Alkalimetallphenolaten eingesetzt werden. Als Beispiel für ein solches Hydrat sei Natriumphenolattrihydrat genannt. Die Menge an zugesetztem Wasser ist jedoch vorzugsweise so bemessen, daß pro Mol Base maximal 5 Mol Wasser eingesetzt werden. Höhere Wasserkonzentrationen führen i.a. zu schlechteren Umsätzen und Zersetzung gebildeter Carbonate. Als organische Basen seien, ohne das erfindungsgemäße Verfahren einzuschränken, tertiäre Amine, die als organische Reste C₆-C₁₀-Aryl, C₇-C₁₂-Aralkyl- und/oder C₁-C₂₀-Alkyl-Reste tragen können oder Pyridinbasen oder hydrierte Pyridinbasen darstellen, genannt, beispielsweise Triethylamin, Tripropylamin, Tributylamin, Trioctylamin, Benzyldimethylamin, Dioctylbenzylamin, Dimethylphenethylamin, 1-Dimethylamino-2-phenylpropan, Pyridin, N-Methylpiperidin, 1,2,2,6,6-Pentamethylpiperidin. Bevorzugt wird als Base ein Alkalisalz einer aromatischen Hydroxyverbindung verwendet, besonders bevorzugt ein Alkalisalz der aromatischen Hydroxyverbindung, die auch zum organischen Carbonat umgesetzt werden soll. Diese Alkalisalze können Lithium-, Natrium-, Kalium-, Rubidium- oder Cäsiumsalze sein. Bevorzugt werden Lithium-, Natrium-und Kaliumphenolat, besonders bevorzugt Natriumphenolat eingesetzt.

Die Base kann dem Reaktionsgemisch als reine Verbindung in fester Form oder als Schmelze zugesetzt werden. In einer weiteren Ausführungsform der Erfindung wird die Base dem Reaktionsgemisch als Lösung, die 0,1 bis 80 Gew.-%, bevorzugt 0,5 bis 65 Gew.-%, besonders bevorzugt 1 bis 50 Gew.-% der Base enthält, zugesetzt. Als Lösungsmittel können hierbei sowohl Alkohole oder Phenole, wie z.B. das umzusetzende Phenol, als auch inerte Lösungsmittel verwendet werden. Als Beispiele für Lösungsmittel seien Dimethylacetamid, N-Methylpyrrolidinon, Dioxan, t-Butanol, Cumylalkohol, Isoamylalkohol, Tetramethylharnstoff, Diethylenglykol, halogenierte Kohlenwasserstoffe (z.B. Chlorbenzol oder Dichlorbenzol) und Ether genannt. Diese Lösungsmittel können allein oder in beliebiger Kombination miteinander eingesetzt werden. So besteht eine Ausführungsform des erfindungsgemäßen Verfahrens beispielsweise darin, daß man die Base in einer Phenolschmelze löst, die mit einem Lösungsmittel verdünnt wurde. Bevorzugt wird die Base in der Schmelze einer aromatischen Hydroxyverbindung gelöst, besonders bevorzugt in einer Schmelze der aromatischen Hydroxyverbindung, die zum organischen Carbonat umgesetzt werden soll. Ganz besonders bevorzugt wird die Base, in Phenol gelöst, zugesetzt.

Die Base wird in einer von der Stöchiometrie unabhängigen Menge zugesetzt. Das Verhältnis von Palladium zu Base wird vorzugsweise so gewählt, daß pro Mol Palladium 0,1 bis 500, bevorzugt 0,5 bis 200, besonders bevorzugt 0,9 bis 130 Äquivalente Base eingesetzt werden.

Das erfindungsgemäße Verfahren wird vorzugsweise ohne Lösungsmittel durchgeführt. Selbstverständlich können auch inerte Lösungsmittel verwendet werden. Als solche seien die im Zusammenhang mit der Base oben erwähnten genannt.

Die für das erfindungsgemäße Verfahren geeigneten Edelmetall-Katalysatoren bestehen aus mindestens einem Metall der Gruppe VIII, vorzugsweise Palladium. Das Edelmetall kann z.B. bei dem erfindungsgemäßen Verfahren in verschiedener Form zugegeben werden. Palladium kann in metallischer Form oder bevorzugt in Form von Palladium-Verbindungen der Oxidationsstufen 0 und +2, wie beispielsweise Palladium(II)-acetylacetonat, -halogenid, -carboxylat von C₂-C₆-Carbonsäuren, -nitrat, -oxid oder Palladiumkomplexe, die beispielsweise Olefine, Amine, Phosphine und Halogenide enthalten können, eingesetzt werden. Besonders bevorzugt sind Palladiumbromid und Palladiumacetylacetonat.

Die Menge an Edelmetall-Katalysator ist im erfindungsgemäßen Verfahren nicht beschränkt. Bevorzugt wird so viel Katalysator zugesetzt, daß seine Konzentration, gerechnet als Metall, im Reaktionsansatz 10 bis 3.000 ppm beträgt, bevorzugt sind Konzentrationen von 10 bis 1.000 ppm, besonders bevorzugt 50 bis 1.000 ppm.

Die erzielbaren Katalysezyklen, d.h. die gebildeten Mole Diarylcarbonat pro Mol Palladium und Stunde, liegen bei 1 bis 50.000, bevorzugt 100 bis 30.000.

Als Cokatalysator für das erfindungsgemäße Verfahren wird eine Metallverbindung der Gruppen III A, IV A, V A, I B, II B, VI B oder VII B des Periodensystems der Elemente (Mendelejew) eingesetzt, wobei das Metall in verschiedenen Oxidationsstufen eingesetzt werden kann. Ohne das erfindungsgemäße Verfahren einzuschränken, seien Mangan(II), Mangan(III), Kupfer(I), Kupfer(II), Kobalt(II), Kobalt(III), Vanadium(III) und Vanadium(IV) genannt. Die Metalle können beispielsweise als Halogenide, Oxide, Carboxylate von C₂-C₆-Carbonsäuren, Diketonate oder Nitrate sowie als Komplexverbindungen, die beispielsweise Kohlenmonoxid, Olefine, Amine, Phosphine und Halogenide enthalten können, eingesetzt werden. Bevorzugt werden Manganverbindungen im erfindungsgemäßen Verfahren verwendet, besonders bevorzugt Mangan(II)komplexe, ganz besonders bevorzugt Mangan(II)acetylacetonat.

Der Cokatalysator wird in einer Menge zugesetzt, daß seine Konzentration im Bereich von 0,001 bis 20 Gew.-% des Reaktionsgemisches liegt, bevorzugt ist der Konzentrationsbereich von 0,005 bis 5 Gew.-%, besonders bevorzugt 0,01 bis 2 Gew.-%.

Bei den im Rahmen der vorliegenden Erfindung eingesetzten quartären Salzen kann es sich beispielsweise um mit organischen Resten substituierte Ammonium-oder Phosphoniumsalze handeln. Geeignet für den Einsatz im erfindungsgemäßen Verfahren sind Ammonium- und Phosphoniumsalze, die als organische Reste C₆-C₁₀-Aryl-, C₇-C₁₂-Aralkyl- und/oder C₁-C₂₀-Alkyl-Reste und als Anion ein Halogenid, Tetrafluoroborat oder Hexafluorophosphat tragen. Bevorzugt werden in das erfindungsgemäße Verfahren Ammoniumsalze, die als organische Reste C₆-C₁₀-Aryl-, C₇-C₁₂-Aralkyl- und/oder C₁-C₂₀-Alkyl-Reste und als Anion ein Halogenid tragen, eingesetzt, besonders bevorzugt ist Tetrabutylammoniumbromid.

Die Menge eines solchen quartären Salzes beträgt 0,1 bis 50 Gew.-%, bezogen auf das Gewicht des Reaktionsgemisches. Vorzugsweise beträgt diese Menge 0,5 bis 15 Gew.-%, besonders bevorzugt 1 bis 5 Gew.-%.

Das erfindungsgemäße Verfahren wird, vorzugsweise ohne Lösungsmittel, bei 30 bis 200°C, bevorzugt bei 30 bis 150°C, besonders bevorzugt bei 40 bis 120°C bei einem Druck von 1 bis 100 bar, bevorzugt 2 bis 50 bar, besonders bevorzugt bei 5 bis 25 bar durchgeführt.

Die nach dem erfindungsgemäßen Verfahren erzielbaren Raum-Zeit-Ausbeuten an Diarylcarbonat, d.h. die gebildete Diarylcarbonatmenge in g pro Liter Reaktorvolumen und Stunde, liegen bei 1 bis 3.500 g/lh, bevorzugt 10 bis 2.500 g/lh.

Die Reproduzierbarkeit wird durch die im folgenden beschriebene Vorgehensweise erreicht. Der Edelmetall-Katalysator wird vor der Reaktion aktiviert. Dazu wird die Edelmetallverbindung, so bemessen, daß die Konzentration des Edelmetalls im Aktivierungsansatz 0,0001 bis 30 Gew.-%, bevorzugt 0,001 bis 10 Gew.-% beträgt, und in einem inerten Lösungsmittel oder direkt in der Schmelze der aromatischen Hydroxyverbindung oder Mischungen derselben gelöst. Zu dieser Lösung wird ein quartäres Salz gegeben, bei dem es sich um die oben beschriebenen, mit organischen Resten substituierte Ammonium- oder Phosphoniumsalze handelt. Diese Lösung wird anschließend bei 15 bis 200°C, bevorzugt bei 20 bis 150°C, besonders bevorzugt bei 40 bis 100°C mit Kohlenmonoxid behandelt. Das kann sowohl dadurch geschehen, daß man bei Normaldruck pro Gramm des eingesetzten Edelmetalls Kohlenmonoxid in einer Menge von 0,1 bis 250 l/h, bevorzugt 0,5 bis 200 l/h, besonders bevorzugt 1 bis 100 l/h einleitet, als auch dadurch, daß man die Lösung in einem Autoklaven unter einem Druck von 1 bis 300 bar, bevorzugt 1 bis 200 bar, besonders bevorzugt 1 bis 150 bar mit Kohlenmonoxid versetzt. Die Aktivierungszeit hängt vom verwendeten Edelmetallkatalysator und von einem gegebenenfalls eingesetzten inerten Lösungsmittel ab. Sie beträgt im allgemeinen wenige Minuten bis einige Stunden, beispielsweise 0,05 bis 5 h, bevorzugt 0,1 bis 3 h, besonders bevorzugt 0,25 bis 2 h. Der Edelmetall-Katalysator kann direkt vor der Reaktion aktiviert, aber auch nach Abtrennung des Lösungsmittel oder der aromatischen Hydroxyverbindung, z.B. durch Abdestillieren, isoliert und ohne Aktivitätsverlust gelagert werden.

Als Reaktoren für das erfindungsgemäße Verfahren sind Rührkessel, Autoklaven und Blasensäulen geeignet, wobei diese als Einzelreaktoren oder als Kaskade eingesetzt werden können. In einer Kaskade können 2 bis 15, bevorzugt 2 bis 10, besonders bevorzugt 2 bis 5 Reaktoren hintereinander geschaltet sein.

Zur Vermischung der erfindungsgemäß zu verwendenden Reaktionskomponenten sind die Rührbehälter mit dafür brauchbaren Rührer ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und verschiedene Hohlrührertypen. Bevorzugte Rührer sind solche, die eine effektive Vermischung von Gasen und Flüssigkeiten erlauben, beispielsweise Hohlrohrbegasungsrührer, Propellerrührer etc. Als Blasensäulen können in dem erfindungsgemäßen Verfahren folgende Typen eingesetzt werden: einfache Blasensäulen, Blasensäulen mit Einbauten, wie z.B.: Blasensäulen mit parallelen Kammern, Kaskaden-Blasensäulen mit Siebböden oder Einlochböden, Blasensäulen mit Packungen, mit statischen Mischern, pulsierende Siebbodenblasensäulen, Schlaufenreaktoren wie z.B.: Mammutschlaufenreaktoren, Abstromschlaufenreaktoren, Strahlschlaufenreaktor, Freistrahlreaktoren, Strahldüsenreaktoren, Blasensäulen mit Flüssigkeitstauchstrahler, Abstrom-Aufstrom-Blasensäulen und weitere dem Fachmann bekannte Blasensäulenreaktoren (H. Gerstenberg, Chem. Ing. Techn. 61 (1979) Nr. 3, S. 208-216; W.-D. Deckwer, Reaktionstechnik in Blasensäulen, Otto Salle Verlag 1985).

In der bevorzugten Ausführungsform kommen Blasensäulenreaktoren und Blasensäulen Kaskaden zum Einsatz, die eine effektive Vermischung von Gas und Flüssigkeiten erlauben, wie beispielsweise Kaskaden-Blasensäulen und Schlaufenreaktoren. Zur Aufrechterhaltung einer guten Durchmischung von Flüssigkeit und Reaktionsgas können Verteilungs- und Redispergierorgane entlang der Längsachse der Blasensäulenreaktoren angebracht sein. Als feste Redispergierorgane kommen Einlochböden, Lochplatten, Sieböden, sowie weitere dem Fachmann bekannte Einbauten zum Einsatz.

Für die Erstdispergierung des Reaktionsgases in der flüssigen Phase bei der Eindosierung sind übliche Vorrichtungen wie poröse Sinterplatten, Lochplatten, Siebböden, Einsteckrohre, Düsen, Begasungsringe und weitere dem Fachmann bekannte Dispergiervorrichtungen einsetzbar.

Das erfindungsgemäße Verfahren kann in verschiedenen Ausführungsvarianten ausgeübt werden. Eine Möglichkeit besteht in der diskontinuierlichen Durchführung. Dabei werden CO und Sauerstoff entweder durch einen Begasungsrührer, im Falle eines Rührkessels oder andere bekannte Gasverteilungsorgane in die Reaktionsmischung geleitet. Durch das überschüssige Reaktionsgas wird das Reaktionswasser kontinuierlich aus dem Reaktor entfernt. Nach Erreichen des vorgesehenen Umsatzes wird das Reaktionsgemisch aus dem Reaktor entfernt oder gegebenenfalls im Reaktor aufgearbeitet.

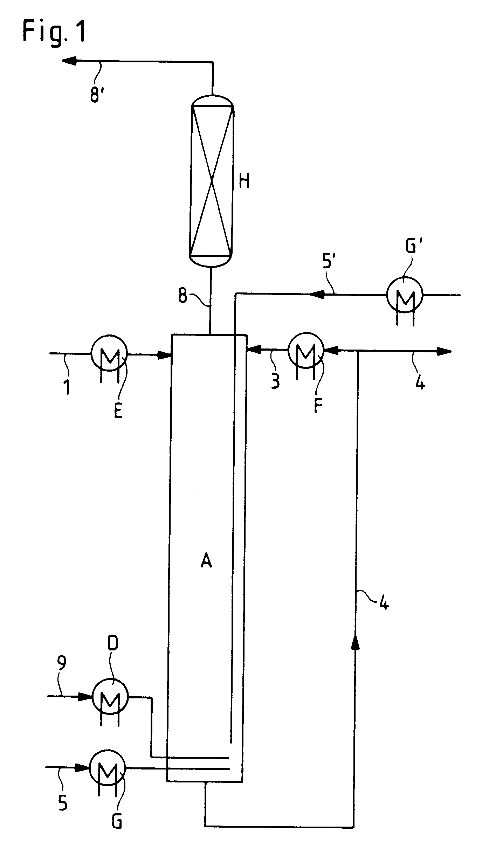

In den bevorzugten Ausführungsformen des erfindungsgemäßen Verfahrens kommt eine kontinuierliche Arbeitweise im Einzelreaktor oder in einer Kaskade aus Reaktoren zum Einsatz. In den Figuren 1, 2 und 3 sind beispielhaft Arbeitsweisen mit einem bzw. drei Reaktoren (A, B und C) dargestellt, wobei die erfindungsgemäße Arbeitsweise nicht auf diese Beispiele eingeschränkt werden soll.

Die bevorzugte Ausführungsform des erfindungsgemäßen Verfahrens besteht darin, daß man den Edelmetall-Katalysator in der oben beschriebenen Weise aktiviert und diese Lösung dann gleichzeitig mit einer weiteren Lösung, die die restlichen Komponenten des Reaktionssystems in den oben angegebenen Konzentrationen enthält, kontinuierlich (über Leitung (1) in Figur 1) dem Reaktor (A) zusetzt. Durch ein vorgeschaltetes Erhitzerelement (E) können die flüssigen Reaktionskomponenten gegebenenfalls auf die vorgesehene Reaktionstemperatur vorgeheizt werden. Die aus dem Reaktoren zu entnehmende Flüssigphase wird am unteren Reaktorende entnommen und über die Leitungen (4) der Aufarbeitung zugeführt. Die Regelung des gewünschten Füllstandes im kontinuierlich betriebenen Reaktor erfolgt nach dem Stand der Technik. Gegebenenfalls kann ein Teilstrom über Leitung (3) wieder in den Reaktor (A) zurückgeführt werden. Durch ein vorgeschaltetes Erhitzerelement (F) können die flüssigen Reaktionskomponenten gegebenenfalls wieder auf die vorgesehene Reaktionstemperatur vorgeheizt werden.

Das aus Kohlenmonoxid, Sauerstoff und gegebenenfalls Inertgas bestehende Reaktionsgas kann über die Leitung (5) oder (5') am unteren Ende des Reaktors (A) eingeleitet und gegebenenfalls zuvor durch einen Vorerhitzer (G) bzw. (G') auf die Reaktionstemperatur vorgeheizt werden. Die Sauerstoffdosierung kann hierbei unabhängig von CO und Inertgas bzw. zusammen mit Inertgas erfolgen. Im Falle einer separaten Sauerstoffdosierung erfolgt diese durch Leitung (9) und Vorerhitzer (D). Dabei werden CO und Sauerstoff, in den oben angegebenen Mengen, entweder durch einen Begasungsrührer, im Falle einer Rührkessels, oder andere bekannte Gasverteilungsorgane in der Reaktionsmischung verteilt. Das überschüssige Reaktionsgas verläßt zusammen mit dem Reaktionswasser und mitgerissenem Edukt (II) über Leitung (8) den Reaktor. Im Trennorgan (H) wird der größte Teil des Edukts (II) abgetrennt und in den Reaktor (A) zurückgeführt. Über Leitung (8') verläßt das überschüssige Reaktionsgas zusammen mit dem Reaktionswasser den Reaktor. Das Reaktionswasser wird aus dem Reaktionsgas nach dem Stand der Technik entfernt. Das Reaktionsgas wird anschließend zusammen mit dem Ersatz des verbrauchten Reaktionsgases dem Reaktor (A) wieder zugeführt.

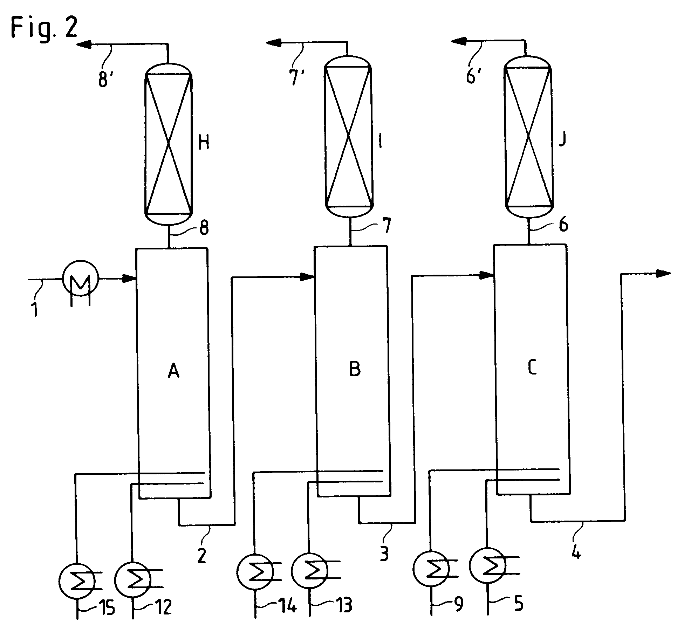

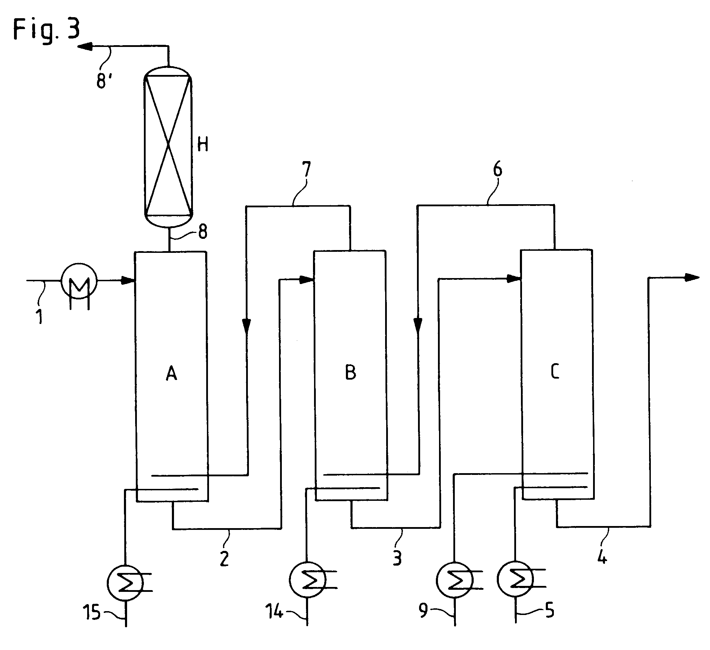

Im Falle der Verwendung einer Reaktorkaskade (Fig. 2 bzw. 3) werden die oben beschriebenen flüssigen Reaktionskomponenten in den ersten Reaktor (A) eindosiert und können gegebenenfalls in einem vorgeschalteten Erhitzerelement auf die vorgesehene Reaktionstemperatur vorgeheizt werden. Sie werden in flüssiger Form über Leitung (1) bevorzugt am oberen Ende des Reaktors eingebracht. Die aus den jeweiligen Reaktoren zu entnehmende Flüssigphase wird am unteren Reaktorende entnommen und über die Leitungen (2) oder (3) in die jeweils folgenden Reaktoren (B) oder (C) am oberen Ende wieder eindosiert. Über Leitung (4) wird der Produktstrom entnommen und der Aufarbeitung zugeführt. Die Regelung des gewünschten Füllstandes in den kontinuierlich betriebenen Reaktoren erfolgt nach dem Stand der Technik. Bei der Benutzung einer Kaskade kann die Gasphase durch den kontinuierlich laufenden Flüssigkeitsstrom entweder im Querstrom (Fig. 2) oder im Gegenstrom (Fig. 3) geschickt werden.

Querstrom bedeutet hierbei daß die Reaktionsgase über die Leitungen (12), (13) und (5) (Fig. 2) eindosiert werden und jeweils am oberen Ende eines jeden Reaktors über die Leitungen (8), (7) und (6) (Fig. 2) zusammen mit dem Reaktionswasser und mitgerissenem Edukt (II) wieder entnommen werden, d.h. das Reaktionsgas durchströmt die Reaktoren quer zur Flußrichtung der Flüssigphase. Im Falle einer separaten Sauerstoffdosierung erfolgt dies über die Leitungen (9), (14) und (15). In den Trennorganen (H), (I) und (J) wird das Edukt (II) abgetrennt und in die jeweiligen Reaktoren zurückgeführt. Über die Leitungen (8'), (7') und (6') verläßt das überschüssige Reaktionsgas zusammen mit dem Reaktionswasser den Reaktor. Das Reaktionswasser wird, nach Vereinigung der Abgasströme, aus dem Reaktionsgas nach dem Stand der Technik entfernt. Das Reaktionsgas wird anschließend zusammen mit dem Ersatz des verbrauchten Reaktionsgases den Reaktoren (A), (B) und (C) wieder zugeführt.

Die Gesamtmenge des eindosierten Reaktionsgases kann beliebig auf die einzelnen Reaktoren aufgeteilt werden. In jedem Reaktor wird bevorzugt die Gegenstromfahrweise von Flüssigphase und Gasphase realisiert.

Gegenstromfahrweise (Fig. 3) bedeutet, daß man die Gasphase in den letzten Reaktor (in Fig. 3 Reaktor C) eindosiert, kontinuierlich gegen die vom ersten Reaktor (A) zum letzten Reaktor (C in Fig. 3) laufende Flüssigphase durch die Leitungen (6) und (7) führt und am jeweils unteren Ende des folgenden Reaktors (B) und (A) wieder einleitet. Im Falle einer separaten Sauerstoffdosierung erfolgt dies über die Leitungen (9), (14) und (15). Die Dosierung und Führung der Flüssigphase in den Reaktoren ist mit der Querstromfahrweise identisch. Am oberen Ende des ersten Reaktors (A in Fig. 3) wird das überschüssige Reaktionsgas zusammen mit dem Reaktionswasser und mitgerissenem Edukt (II) über Leitung (8) entnommen. Im Trennorgan (H) wird der größte Teil des Edukts (II) abgtetrennt und in den Reaktor (A) zurückgeführt. Über die Leitung (8') verläßt das überschüssige Reaktionsgas zusammen mit dem Reaktionswasser den Reaktor. Das Reaktionswasser wird aus dem Reaktionsgas nach dem Stand der Technik entfernt und anschließend zusammen mit dem Ersatz des verbrauchten Reaktionsgases dem Reaktor (C) wieder zugeführt.

Die Aufarbeitung des flüssigen Reaktionsgemisches kann z.B. durch Destillation erfolgen, wobei zunächst die aromatische Hydroxyverbindung abgetrennt und in einem weiteren Schritt das aromatische Carbonat isoliert wird. Die im Rückstand befindlichen Katalysatorkomponenten können zurückgewonnen und recyclisiert werden.

Die folgenden Beispiele verdeutlichen das erfindungsgemäße Verfahren, ohne es jedoch auf diese einzuschränken.

### Beispiel 1:

In einem Autoklaven (1 l) mit Begasungsrührer, Kühler und nachgeschalteter Kühlfalle wurden 0,34 g Palladiumbromid und 8,31 g Tetrabutylammoniumbromid bei 80°C in 450 g Phenol gelöst. Durch diese Lösung wurde zur Aktivierung des Katalysators eine Stunde lang Kohlenmonoxid (3 l/h) geleitet. Dann wurden 0,77 g Mangan(II)acetylacetonat und 2,21 g Natriumphenolat, gelöst in 50 g Phenol, zugegeben und unter Einleitung eines Gasgemisch aus Kohlenmonoxid und Sauerstoff (95:5 Vol.%) der Druck auf 10 bar eingestellt. Die Menge an Gasgemisch, bestehend aus Kohlenmonoxid und Sauerstoff (95:5 Vol.%), wurde auf 400 Nl/h eingestellt. Dem Reaktionsgemisch wurde jede Stunde eine Probe entnommen und gaschromatographisch analysiert. Die Analysen ergaben, daß nach 1 Stunde 8,2 % Diphenylcarbonat, nach 2 Stunden 13,75 % Diphenylcarbonat und nach 3 Stunden 18,6 % Diphenylcarbonat im Reaktionsgemisch enthalten waren. In der Kühlfalle waren 8,75 g eines Phenol/Wasser-Gemisches kondensiert.
- Katalysezyklen pro Stunde :: 113 (Durchschnitt)
- Raum-Zeit-Ausbeute :: 31 g/l·h (Durchschnitt)

Der Katalysezyklus bedeutet hierbei Mol gebildetes Diphenylcarbonat pro Mol Pd-Verbindung pro Zeiteinheit.

### Vergleichsbeispiel 1:

Der Versuch wurde wie in Beispiel 1 beschrieben wiederholt, jedoch wurden statt 400 Nl/h nur 6 Nl/h des Kohlenmonoxid/Sauerstoff-Gasgemisches eingeleitet. Die gaschromatographische Analyse der Proben ergab, daß nach einer Stunde 4,8 % Diphenylcarbonat im Reaktionsgemisch enthalten waren. Nach 2 Stunden waren 5,3 % Diphenylcarbonat und nach 3 Stunden 5,4 % Diphenylcarbonat im Reaktionsgemisch enthalten. In der Kühlfalle waren 0,2 g eines Phenol/Wasser-Gemisches kondensiert.

### Beispiel 2:

In einem Blasensäulenreaktor (5 cm Durchmesser und 50 cm Höhe) mit Kühler und nachgeschalteter Kühlfalle wurden 0,34 g Palladiumbromid und 8,31 g Tetrabutylammoniumbromid bei 80°C in 450 g Phenol gelöst. Durch diese Lösung wurde zur Aktivierung des Katalysators eine Stunde lang Kohlenmonoxid (3 l/h) geleitet. Nach erfolgter Aktivierung wurden 0,77 g Mangan(II)acetylacetonat und 2,21 g Natriumphenolat, gelöst in 50 g Phenol, zugegeben und bei 10 bar ein Gasgemisch (600 Nl/h) bestehend aus Kohlenmonoxid und Sauerstoff (95:5 Vol.%) eingeleitet. Dem Reaktionsgemisch wurde jede Stunde eine Probe entnommen und gaschromatographisch analysiert. Die Analysen ergaben, daß nach einer Stunde 9,6 % Diphenylcarbonat, nach 2 Stunden 17,2 % Diphenylcarbonat und nach 3 Stunden 23,0 % Diphenylcarbonat im Reaktionsgemisch enthalten waren. In der Kühlfalle waren 11,3 g eines Phenol/Wasser-Gemischs kondensiert.
- Katalysezyklen pro Stunde :: 143 (Durchschnitt)
- Raum-Zeit-Ausbeute :: 38,3 g/l·h (Durchschnitt)

### Beispiel 3

Das Beispiel 2 wurde wiederholt, wobei statt 0,34 g nur 0,08 g Palladiumbromid eingesetzt wurden und die Reaktionsdurchführung bei 8 bar und 300 Nl Gasgemisch erfolgte. Der Palladiumbromidüberschuß wurde in diesem Beispiel reduziert. Mangan(II)acetylacetonat wurde bereits vor der Aktivierung des Palladiums zugesetzt.

Dem Reaktionsgemisch wurde stündlich eine Probe entnommen und gaschromatographisch analysiert. Die Analysen ergaben, daß nach einer Stunde 12,5 % Diphenylcarbonat, nach 2 Stunden 16,2 % Diphenylcarbonat und nach 3 Stunden 21,5 % Diphenylcarbonat im Reaktionsgemisch enthalten waren. In der Kühlfalle waren 12,1 g eines Phenol/Wasser-Gemisches kondensiert.
- Katalysezyklen pro Stunde:: Ø 486 (Durchschnitt)
- Raum-Zeit-Ausbeute:: 37,5 g/lh (Durchschnitt)

## Patentansprüche

1. Verfahren zur Herstellung eines organischen Carbonats der Formel (I)
R-O-CO-O-R (I)
in der
R ein gegebenenfalls substituiertes C₆-C₁₂-Aryl, bevorzugt ein gegebenenfalls substituiertes C₆-Aryl, besonders bevorzugt Phenyl bedeutet,
durch Umsetzung einer aromatischen Hydroxyverbindung der Formel (II),
R-O-H (II),
worin
R die oben angegebene Bedeutung hat,
mit Kohlenmonoxid und Sauerstoff in Gegenwart eines aktivierten Edelmetall-Katalysators, der in Gegenwart eines quartären Salzes durch Behandlung mit Kohlenmonoxid in flüssiger Phase bei erhöhter Temperatur aus einer Verbindung eines Edelmetalls der Gruppe VIIIb des Periodensystems der Elemente (Mendelejew) hergestellt wurde, eines Cokatalysators, eines quartären Salzes und einer Base, dadurch gekennzeichnet, daß man das Reaktionswasser durch überschüssiges Reaktionsgas aus dem Reaktionsgemisch entfernt.

2. Verfahren nach Anspruch 1, dadurch gekennzeichnet, daß man das Reaktionsgas in einer Menge von 1 bis 100.000 Nl pro Liter Reaktionslösung, bevorzugt 5 bis 50.000 Nl pro Liter Reaktionslösung und besonders bevorzugt 10 bis 10.000 Nl pro Liter Reaktionslösung einleitet.

3. Verfahren nach Anspruch 1 und 2, dadurch gekennzeichnet, daß man als Reaktionsgas ein Gemisch aus Kohlenmonoxid, Sauerstoff und einem Inertgas verwendet, wobei das Inertgas bevorzugt mit Wasser ein Azeotrop bilden und dessen Konzentration im Reaktionsgas 0-30 Vol.%, bevorzugt 0-15 Vol.%, besonders bevorzugt 0-5 Vol.% beträgt.

4. Verfahren nach den Ansprüchen 1 bis 3, dadurch gekennzeichnet, daß man als Reaktoren Rührkessel oder Blasensäulen verwendet, wobei diese als Einzelreaktoren oder als Kaskade eingesetzt werden können.

5. Verfahren nach Anspruch 4, dadurch gekennzeichnet, daß in einer Reaktorkaskade 2 bis 15, bevorzugt 2 bis 10, besonders bevorzugt 2 bis 5 Reaktoren hintereinander geschaltet werden.

6. Verfahren nach den Ansprüchen 1 bis 5, dadurch gekennzeichnet, daß das Edelmetall Palladium ist, bevorzugt in Form von Palladiumverbindungen, besonders bevorzugt in Form von Palladiumbromid.

7. Verfahren nach den Ansprüchen 1 bis 6, dadurch gekennzeichnet, daß man als organische Hydroxyverbindung Phenol einsetzt.

8. Verfahren nach den Ansprüchen 1 bis 7, dadurch gekennzeichnet, daß man als Base ein tertiäres Amin, Alkaliphenolat oder Alkalisalz schwacher Säuren, vorzugsweise Alkalicarboxylate und/oder -phenolate, besonders bevorzugt Natriumphenolat verwendet.

9. Verfahren nach den Ansprüchen 1 bis 8, dadurch gekennzeichnet, daß man als quartäres Salz Tetraalkylammonium- oder -phosphoniumsalze, bevorzugt Tetraalkylammoniumsalze, besonders bevorzugt Tetrabutylammoniumbromid einsetzt.

10. Verfahren nach den Ansprüchen 1 bis 9, dadurch gekennzeichnet, daß man es bei 30 bis 200°C, bevorzugt 30 bis 150°C, besonders bevorzugt 40 bis 120°C, bei einem Druck von 1 bis 100 bar, bevorzugt 2 bis 50 bar, besonders bevorzugt 5 bis 25 bar und unter Rühren durchführt.

## Claims

1. Process for the preparation of an organic carbonate of formula (I)
R-O-CO-O-R (I)
in which
R denotes an optionally substituted C₆-C₁₂ aryl, preferably an optionally substituted c₆ aryl, particularly preferably phenyl,
by reacting an aromatic hydroxy compound of formula (II),
R-O-H (II),
in which
R has the above meaning,
with carbon monoxide and oxygen in the presence of an activated precious metal catalyst, which was prepared in the presence of a quaternary salt by treating with carbon monoxide in the liquid phase at elevated temperature from a compound of a precious metal from group VIIIb of the periodic table (Mendeleev), a co-catalyst, a quaternary salt and a base, characterised in that the water of reaction is removed from the reaction mixture by excess reaction gas.

2. Process according to claim 1, characterised in that the reaction gas is introduced in a quantity of 1 to 100 000 Nl per litre of reaction solution, preferably 5 to 50 000 Nl per litre of reaction solution and particularly preferably 10 to 10 000 Nl per litre of reaction solution.

3. Process according to claim 1 or 2, characterised in that a mixture of carbon monoxide, oxygen and an inert gas is used as the reaction gas, the inert gas preferably forming an azeotype with water and its concentration in the reaction gas being 0-30 vol.%, preferably 0-15 vol.%, particularly preferably 0-5 vol.%.

4. Process according to claims 1 to 3, characterised in that agitated vessels or bubble columns are used as the reactors, and these may be used as individual reactors or as a cascade.

5. Process according to claim 4, characterised in that 2 to 15, preferably 2 to 10, particularly preferably 2 to 5 reactors are connected in series in a reactor cascade.

6. Process according to claims 1 to 5, characterised in that the precious metal is palladium, preferably in the form of palladium compounds, particularly preferably in the form of palladium bromide.

7. Process according to claims 1 to 6, characterised in that phenol is used as the organic hydroxy compound.

8. Process according to claims 1 to 7, characterised in that a tertiary amine, alkali phenolate or alkali metal salt of weak acids, preferably alkali metal carboxylates and/or phenolates, particularly preferably sodium phenolate, is used as the base.

9. Process according to claims 1 to 8, characterised in that tetraalkylammonium or tetraalkylphosphonium salts, preferably tetraalkylammonium salts, particularly preferably tetrabutylammonium bromide is used as the quaternary salt.

10. Process according to claims 1 to 9, characterised in that it is carried out at 30 to 200°C, preferably 30 to 150°C, particularly preferably 40 to 120°C, under a pressure of 1 to 100 bar, preferably 2 to 50 bar, particularly preferably 5 to 25 bar, and with stirring.

## Revendications

1. Procédé de préparation d'un carbonate organique de formule (I)
R-O-CO-O-R (I)
dans laquelle
R représente un groupe aryle en C₆-C₁₂ éventuellement substitué, de préférence un groupe aryle en C₆ éventuellement substitué et plus spécialement le groupe phényle,
par réaction d'un composé aromatique hydroxylé de formule (II)
R-O-H (II)
dans laquelle
R a les significations indiquées ci-dessus,
avec le monoxyde de carbone et l'oxygène en présence d'un catalyseur à base de métal noble activé qui a été préparé à partir d'un composé d'un métal noble du groupe VIIIb de la classification périodique des éléments (Mendeleïev) en présence d'un sel quaternaire par traitement à l'aide du monoxyde de carbone en phase liquide à température élevée, d'un catalyseur auxiliaire, d'un sel quaternaire et d'une base, caractérisé en ce que l'on élimine l'eau formée dans la réaction du mélange de réaction à l'aide de l'excès des gaz de réaction.

2. Procédé selon la revendication 1, caractérisé en ce que l'on injecte les gaz de réaction en quantité de 1 à 100 000 l normaux par litre de la solution de réaction, de préférence de 5 à 50 000 l normaux par litre de la solution de réaction et plus spécialement de 10 à 10 000 l normaux par litre de la solution de réaction.

3. Procédé selon les revendications 1 et 2, caractérisé en ce que l'on utilise en tant que gaz de réaction un mélange de monoxyde de carbone, d'oxygène et d'un gaz inerte, ce dernier formant de préférence un azéotrope avec l'eau et sa concentration dans les gaz de réaction représentant de 0 à 30 % en volume, de préférence de 0 à 15 % en volume et plus spécialement de 0 à 5 % en volume.

4. Procédé selon les revendications 1 à 3, caractérisé en ce que l'on utilise en tant que réacteurs des récipients équipés de dispositifs d'agitation ou des colonnes à bulles, ces réacteurs pouvant être mis en oeuvre sous forme de réacteurs individuels ou d'une série de réacteurs.

5. Procédé selon la revendication 4, caractérisé en ce que, dans une série de réacteurs, on place successivement 2 à 15, de préférence 2 à 10 et plus spécialement 2 à 5 réacteurs.

6. Procédé selon les revendications 1 à 5 caractérisé en ce que le métal noble est le palladium, de préférence sous la forme de composés du palladium et plus spécialement sous la forme de bromure de palladium.

7. Procédé selon les revendications 1 à 6 caractérisé en ce que le composé organique hydroxylé est le phénol.

8. Procédé selon l'une des revendications 1 à 7 caractérisé en ce que la base utilisée est une amine tertiaire, un phénolate alcalin ou un sel alcalin d'acide faible, de préférence un carboxylate et/ou un phénolate de métal alcalin, et plus spécialement le phénolate de sodium.

9. Procédé selon les revendications 1 à 8 caractérisé en ce que l'on utilise en tant que sels quaternaires des sels de tétraalkylammonium ou de tétraalkylphosphonium, de préférence des sels de tétraalkylammonium et tout spécialement le bromure de tétrabutylammonium.

10. Procédé selon les revendications 1 à 9 caractérisé en ce que l'on opère à des températures de 30 à 200°C, de préférence de 30 à 150°C, plus spécialement de 40 à 120°C, sous des pressions de 1 à 100 bar, de préférence de 2 à 50 bar et plus spécialement de 5 à 25 bar, et sous agitation.
